**Europäisches Patentamt**

**European Patent Office**

**Office européen des brevets**

(19)

(11) Veröffentlichungsnummer: **0 103 677**

**B1**

(12)

# EUROPÄISCHE PATENTSCHRIFT

(45) Veröffentlichungstag der Patentschrift:
13.08.86

(51) Int. Cl.⁴: **C 12 Q 1/68**

(21) Anmeldenummer: **83105453.1**

(22) Anmeldetag: **01.06.83**

(54) **Verfahren zur massenspektroskopischen Sequenzanalyse eines gegebenenfalls modifizierten Oligoribonukleotids oder Oligodesoxyribonukleotids.**

(30) Priorität: 02.06.82 DE 3220733
11.04.83 DE 3312929

(43) Veröffentlichungstag der Anmeldung:
28.03.84 Patentblatt 84/13

(45) Bekanntmachung des Hinweises auf die Patenterteilung:
13.08.86 Patentblatt 86/33

(84) Benannte Vertragsstaaten:
AT BE CH DE FR GB IT LI NL SE

(56) Entgegenhaltungen:
DE-A-2 241 513

NATURE, Band 274, Nr. 5666, 6. Juli 1978 J. STANLEY et al. "A different approach to RNA sequencing", Seiten 87-89
Chemical Abstracts Band 96, Nr. 19, 10. Mai 1982, Columbus, Ohio, USA C. HIGNITE "Nucleic acids and +erivatives mass spectrometry", Abstract Nr. 158397b
Chemical Abstracts Band 96, Nr. 19, 10. Mai 1982, Columbus, Ohio, USA W. ENS et al. "Secondary ion mass spectrometry of protected diribonucleoside monophosphates with a time-flight mass spectrometer", Seite 383, Spalte 2, Abstract Nr. 158572e
Chemical Abstracts Band 92, Nr. 21, 26. Mai 1980, Columbus, Ohio, USA C.J. McNEIL et al.

(73) Patentinhaber: **Gesellschaft für Biotechnologische Forschung mbH (GBF), Mascheroder Weg 1, D-3300 Braunschweig- Stöckheim (DE)**

(72) Erfinder: **Blöcker, Helmut, Dr., Schinkelstrasse 6, D-2000 Hamburg 60 (DE)**
Erfinder: **Frank, Ronald, Dr., Leibnizstrasse 8, D-3340 Wolfenbüttel (DE)**
Erfinder: **Grotjahn, Lutz, Dr., Bocksbergweg 7, D-3300 Braunschweig (DE)**

(74) Vertreter: **Boeters, Hans Dietrich, Dr., Boeters, Bauer & Partner Thomas- Wimmer- Ring 14, D-8000 München 22 (DE)**

(56) Entgegenhaltungen: (Fortsetzung)
"Sequence determination of protected oligodeoxyribonucleotides containing phosphotriester linkages by californium-252 plasma desorption mass spectrometry", Seite 275, Spalte 2, Abst ract Nr. 176839u
Chem. Abstracts 96, Nr. 158572e (1982) und 92, Nr. 176839 u (1980)

# 0 103 677

## Beschreibung

Synthetische Oligonukleotide werden für die modernen Biowissenschaften immer wichtiger. Insbesondere hat die chemische Synthese von vorgegebenen Nukleotidsequenzen die Entwicklung der Gentechnologie gefördert und beschleunigt. Dadurch wurde die Entwicklung neuer und schnellerer Synthesemethoden angeregt, beispielsweise die Entwicklung von besseren Kupplungsmethoden (1, 2), von hochselektiven und milden Entschützungsmitteln (3, 4) und die Verwendung von geeigneten festen Trägern (5, 6, 7). Derartige Synthesen lassen sich heute in einigen Tagen durchführen; demgemäß ist die größere Arbeit bei der sorgfältigen Analyse der entschützten Oligonukleotide zu leisten. Sequenzanalysen werden üblicherweise durch chemische Abbaumethoden (8) oder "Wandering-Spot"-Methoden (9) nach radioaktiver Markierung durchgeführt.

Es ist ein Verfahren (15) bekannt, bei dem voll geschützte Ribonukleotide mit Hilfe eines SIMS-TOF-Massenspektrometers (Sekundärionenflugzeitmassenspektrometer; time-of-flight mass spectrometer = Static SIMS-Massenspektrometer) analysiert werden.

Derartige Massenspektrometer sind kommerziell nicht erhältlich.

Darüberhinaus ist die Präparierung der Proben sehr schwierig und nur von erfahrenen Praktikern durchführbar. Außerdem befriedigt die Genauigkeit der Massenzuordnung bei dem im Stand der Technik verwendeten Massenspektrometer noch nicht. Bei der Bestimmung von Sequenzionen ist die Genauigkeit der Massenzuordnung besonders wichtig, da in vielen Fällen 3'-und 5'-Phosphatsequenzionen mit der gleichen Anzahl von Basen sich nur um wenige Massen voneinander unterscheiden.

Ferner ist ein Verfahren (14) bekannt, bei dem geschützte Oligodesoxyribonukleotide mit einem Californium-252-Plasma-desorptions-Massenspektrometer (Flugzeitmassenspektrometer) analysiert werden. Auch dieses Massenspektrometer ist käuflich nicht zu erwerben. Wiederum ist die Präparierung der Proben sehr schwierig und nur von erfahrenen Praktikern durchführbar. Auch die Genauigkeit der Massenzuordnung ist nach diesem Stand der Technik unbefriedigend.

Schließlich wird für dieses Verfahren angegeben (12), daß Oligonukleotide mit den natürlich auftretenden Phosphodiester-Internukleotidbindungen ein noch komplexeres Fragmentierungsverhalten zeigen und daß die Ausbeute an Molekülionen stark beeinträchtigt wird.

Erfindungsgemäß wird nun ein Verfahren zur massenspektrometrischen Sequenzanalyse eines Oligoribonukleotids oder Oligodesoxyribonukleotids vorgeschlagen, das dadurch gekennzeichnet ist, daß man

(a) das Nukleotid ungeschützt des Massenspektrometrie unterwirft,
(b) die Negativionen registriert und
(c1) die Massendifferenz zwischen gewichtsmäßig aufeinanderfolgenden 5'-P-Ionen und/oder
(c2) die Massendifferenz zwischen gewichtsmäßig aufeinanderfolgenden 3'-P-Ionen ermittelt und
(d) die ermittelten aufeinanderfolgenden Massendifferenzen den Basen zuordnet.

Ferner wird erfindungsgemäß ein Verfahren zur massenspektrometrischen Sequenzanalyse eines modifizierten Oligoribonukleotids oder Oligodesoxyribonukleotids vorgeschlagen, das dadurch gekennzeichnet ist, daß man

(a) das modifizierte Nukleotid einer FAB-Massenspektrometrie oder SIMS-Massenspektrometrie (ausgenommen Static-SIMS-Massenspektrometrie) unterwirft, wobei es sich bei dem modifizierten Nukleotid um eine Nukleotid mit mindestens einem ionischen Phosphatrest (Phosphatladung) oder um ein unter Meßbedingungen einen derartigen Phosphatrest lieferndes Nukleotid handelt,
(b) die Negativionen registriert und
(c1) die Massendifferenz zwischen gewichtsmäßig aufeinanderfolgenden 5'-P-Ionen und/oder
(c2) die Massendifferenz zwischen gewichtsmäßig aufeinanderfolgenden 3'-P-Ionen ermittelt und
(d) die ermittelten aufeinanderfolgenden Massendifferenzen den Basen zuordnet.

Hinsichtlich der FAB-Massenspektrometrie sei beispielsweise auf Barber et al. (10) und Morris et al. (13) hingewiesen. Ein wesentliches Merkmal der FAB-Massenspektrometrie besteht darin, daß das in Form einer Monolayer auf einer Matrix vorliegende zu untersuchende Material mit neutralen, ungeladenen Teilchen bombardiert wird, beispielsweise mit einem Edelgas in einem Feld von beispielsweise 5 kV oder mehr.

Das erfindungsgemäße Verfahren wird also auf ungeschützte bzw. entschützte Oligoribonukleotide oder Oligodesoxyribonukleotide angewandt. Die Obergrenze der Basenanzahl von nach dem erfindungsgemäßen Verfahren zu analysierenden Nukleotiden ist nicht durch das Verfahrensprinzip begrenzt, in der Praxis kann jedoch eine Grenze durch das Auflösungsvermögen des verwendeten Massenspektrographen gesetzt werden.

Richtwerte für die Homogenität des zu sequenzierenden Materiale sind 95, insbesondere 97 und vorzugsweise 99 %. Es muß jedoch dem Fachmann überlassen bleiben und kann ihm zugemutet werden, den geeigneten Homogenitätsgrad zu ermitteln. Es kann sich erweisen, daß von Fall zu Fall ein anderer Homogenitätsgrad wünschenswert ist, da manche Verunreinigungen überproportional im Spektrum vertreten sein können, wie nukleotidisches Material mit lipophilen Gruppen z.B. mit der Tritylgruppe.

Nachstehend wird die Erfindung anhand von Figuren näher erläutert. Es zeigen:

Fig. 1a ein Negativionen-FAB-Massenspektrum des Oligodesoxyribonukleotids a, nämlich d(A-C-T-C-G-A-T-G) (d = desoxy),

Fig. 1b ein Negativionen-FAB-Massenspektrum des Oligodesoxyribonukleotids b, nämlich d(G-C-G-A-T-C-G-C),

Fig. 1c ein Negativionen-FAB-Massenspektrum des Oligodesoxyribonukleotids c, nämlich d(G-A-A-G-A-T-C-T-T-C),

Fig. 2a ein vereinfachtes Strukturschema des Oligodesoxyribonukleotids a,

2

Fig. 2b ein vereinfachtes Strukturschema des Oligodesoxyribonukleotids b,

Fig. 2c ein vereinfachtes Strukturschema des Oligodesoxyribonukleotide c,

Fig. 3 ein vereinfachtee Strukturschema des Oligoribonukleotide d (G-A-U) sowie dessen Negativionen-FAB-Massenspektrum,

Fig. 4 ein vereinfachtes Strukturschema des Oligoribonukleotide d (A-A-A-A-A-A) sowie dessen Negativionen-FAB-Massenspektrum,

Fig. 5 ein vereinfachtes Strukturschema des Oligoribonukleotide d (A-A-A-A-A-A-A-A) sowie dessen Negativionen-FAB-Massenspektrum und

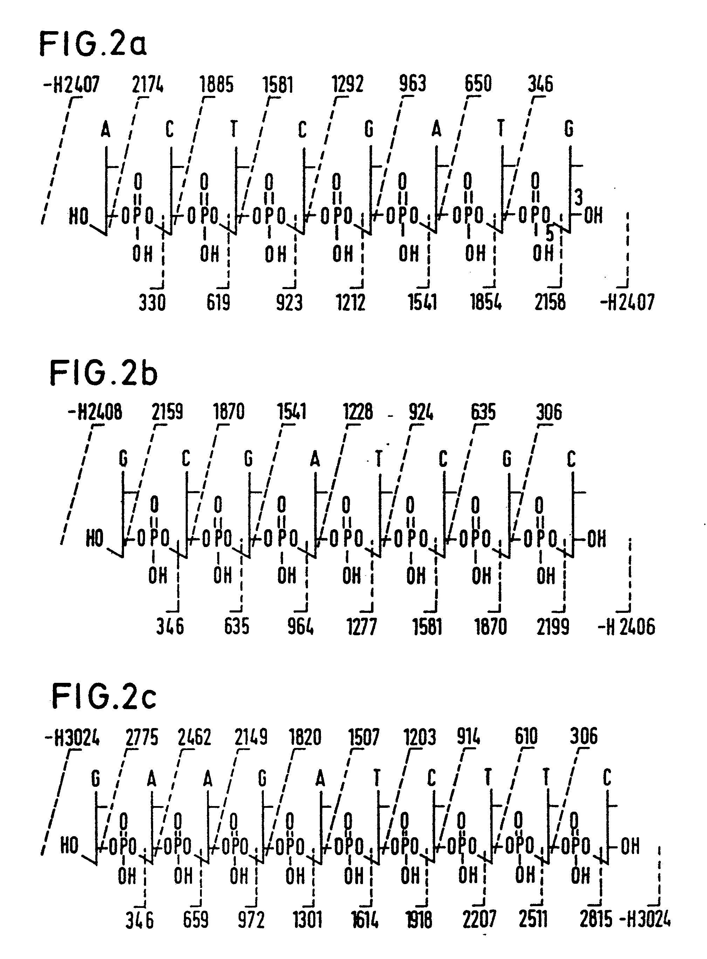

Fig. 6 ein vereinfachtes Strukturschema des Oligodesoxyribonukleotids d (G-A-T) sowie dessen Negativionen-FAB-Massenspektrum,

wobei bei den vereinfachten Strukturschemen der Figuren 2a bis 2c diejenigen Bindungen durch gestrichelte Linien markiert sind, deren Bruch zu den wesentlichen Fragmentionen führt; dabei wurde an den Bruchstellen jeweils die Masse dieser Fragmentionen angeführt, wobei die oben an einer gestrichelten Linie angegebene Masse dem 5'-P-Sequenzion und die unten an einer gestrichelten Linie angegebene Masse dem 3'-P-Sequenzion entspricht.

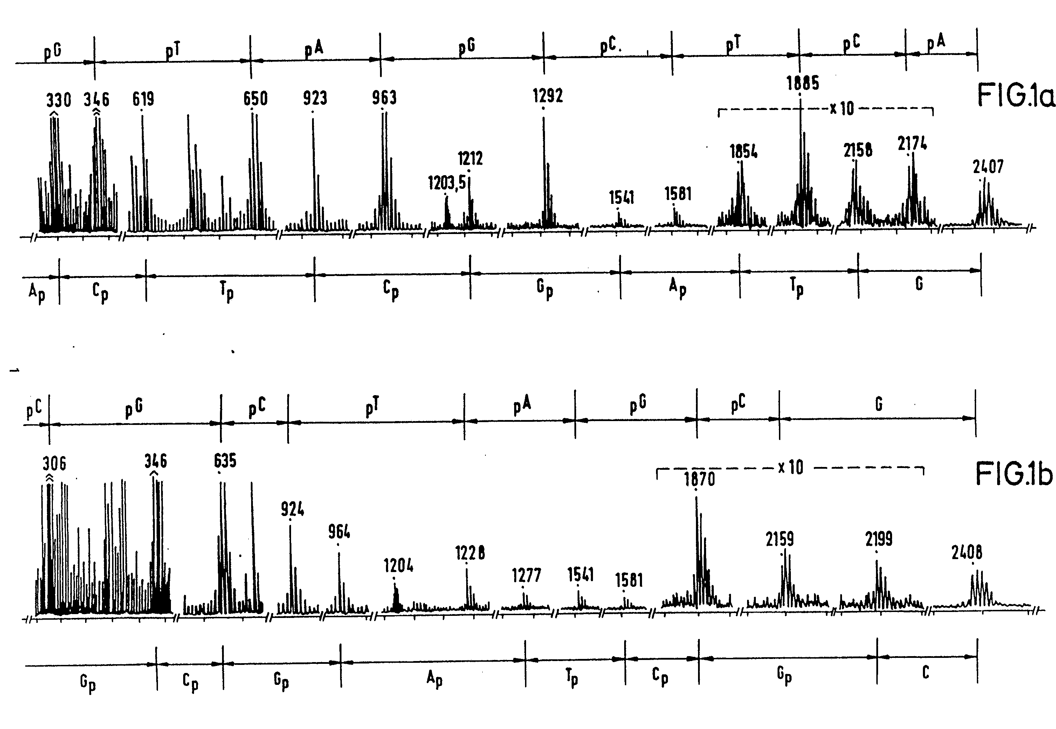

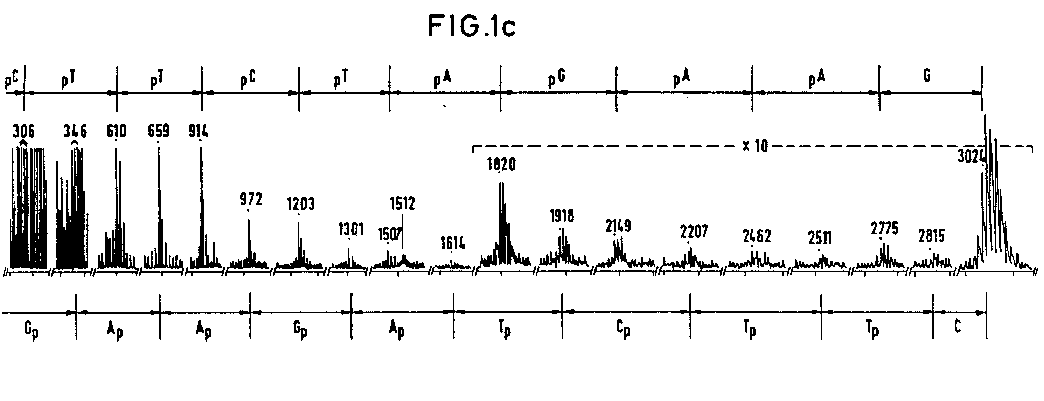

Wenn man reine Oligonukleotide in das erfindungsgemäße Verfahren einsetzt, erhält man praktisch nur spezifische 5'-P-Sequenzionen und 3'-P-Sequenzionen (vgl. Fig. 1a bis 1c). Diese ausschließliche Bildung spezifischer Sequenzionen ermöglicht eine rasche Sequenzanalyse allein dadurch, daß man die Massendifferenzen zwischen aufeinanderfolgenden Sequenzionen des gleichen Typs ermittelt. Eine Unterscheidung zwischen diesen beiden Typen von Sequenzionen ist möglich, da Ionen unterschiedlichen Typs, jedoch mit der gleichen Anzahl von Nukleotideinheiten regelmäßig durch ihre Peakintensität zu unterscheiden sind. Da die Bindung mit dem 3'-0-Atom, das mit einem sekundären Kohlenstoffatom des Zuckeranteile verbunden ist, labiler ist als die Bindung mit dem 5'-0-Atom, das mit einem primären Kohlenstoffatom des Zuckeranteils verbunden ist, treten die 5'-P-Sequenzionen ohne Ausnahme intensiver als die entsprechenden 3'-P-Sequenzionen auf.

Zur Ermittlung der Nukleotidsequenz geht man folgendermaßen vor.

Beginnend vom (M-H)⁻-Peak ((M-H)⁻ = Molekül minus Proton) klassifiziert man alle Sequenzionen entsprechend ihrer Intensität entweder als 5'-P-Ionen oder als 3'-P-Ionen. Für die Figuren 1a und 2a erhält man dabei folgendes Ergebnis:

**Tabelle 1**

| 5'-P-Sequenzionen | 3'-P-Sequenzionen |
|---|---|
| 2407 /(M-H)⁻-Ion/ | |
| 2174 | 2158 |
| 1885 | 1854 |
| 1581 | 1541 |
| 1292 | 1212 |
| 963 | 923 |
| 650 | 619 |
| 346 | 330 |

Danach ermittelt man die genaue Massendifferenz zwischen benachbarten Peaks desselben Sequenzionentyps. Mit der folgenden Tabelle kann man den ermittelten Massendifferenzen das jeweilige Nukleotid zuordnen.

**Tabelle 2**

| Nukleotid | Massendifferenz mittelständig (mit $PO_4H$) | Massendifferenz endständig (ohne $PO_4H$) | (mit $PO_4H$ + OH) |
|---|---|---|---|
| A | 313 | 233 | 330 |
| C | 289 | 209 | 306 |
| G | 329 | 249 | 346 |
| T | 304 | 224 | 321 |

Den Figuren 2a bis 2c kann man entnehmen, daß man hinsichtlich der 5'-P-Sequenzionen und der 3'-P-

3

Sequenzionen zwischen einem 5'-Ende (links in den Figuren) und einem 3'-Ende (rechte in den Figuren) unterscheiden kann, die den entgegengesetzten Enden des jeweiligen Oligodesoxyribonukleotids zugeordnet sind. Dementsprechend kann man die vollständige Basensequenz eines dem erfindungsgemäßen Verfahren unterworfenen Oligonukleotids zweimal ablesen, indem man entweder am 5'-Ende oder am 3'-Ende beginnt.

Diese Möglichkeit, die Basensequenz eines Oligonukleotids in zwei verschiedenen Richtungen abzulesen, bietet die Möglichkeit, daß man die Basensequenz nicht unbedingt in jeder Leserichtung vollständig ablesen muß, sondern nur bis zu bzw. ab einem Überlappungsbereich. Dieses Vorgehen kann dann vorteilhaft sein, wenn sich (beispielsweise infolge von Verunreinigungen des zu untersuchenden Oligonukleotide) die Massen der Sequenzionen mit zunehmender Massenzahl weniger genau ermitteln lassen. In diesem Fall kann man eine vollständige Sequenzanalyse durch Zusammensetzen der in beiden Leserichtungen nur bis zum bzw. ab Überlappungsbereich für die kleineren Massenzahlen gewonnenen Ergebnisse ermitteln.

Es ist klar, daß alle Fragmente derselben Zusammensetzung dieselbe Masse unabhängig davon besitzen, ob es sich um 5'-P-Sequenzionen oder um 3'-P-Sequenzionen handelt. So zeigt das Spektrum der Sequenz b (Fig. 1b) nur einen Peak für beide endständigen Dinukleotidionen. Trotz dieses Umstande bietet die Ermittlung der Sequenz keinerlei Probleme. Aus dem Gesamtspektrum ist klar zu entnehmen, daß in einem weiten Massenbereich nur ein einziger Peak auftritt. Die Massen und die Peakintensitäten der höheren Oligonukleotidionen bestätigen klar, daß (gelesen vom 5'-Ende) die zweite Position C und die siebente Position G und der einzelne Peak der Masse 635 beide Dinukleotidionen repräsentiert.

Synthese der experimentell untersuchten Oligodesoxyribonukleotide

Im wesentlichen arbeitete man nach den bewährten Phosphotriestermethoden. Es wurden Benzoyl-, Anisoyl- und Isobutyryl-Gruppen zum Schutz der heterozyklischen Basen, 4-Methoxytrityl-Gruppen zum Schutz von 5'-OH und 2-Chlorphenyl- und 2,2,2-Tribromäthyl-Gruppen zum Schutz der Phosphatfunktionen verwendet. Die Kondensationsreaktionen wurden in Lösung mit 2,4,6—Triisopropylbenzolsulfonyl-3-nitro-1,2,4-triazolid als Kondensationsmittel durchgeführt. Schließlich bildete man die ungeschützten Oligomeren durch Behandeln mit Pyridin (10 %) in konzentriertem Ammoniak und danach durch Behandeln mit Essigsäure (80 %). Homogenitäten von mehr als 99 % wurden routinemäßig nach Ionenaustauschchromatographie (Sephadex A-25 in Gegenwart von 7 m Harnstoff) und nachfolgendes Entsalzen erhalten. Die Homogenität wurde sorgfältig durch Umkehrphasen-HPLC (Umkehrphasen-Hochdruckflüssigchromatographie), Polyacrylamid-Gelelektrophorese in Gegenwart von Kettenlängen-Markierungen (11) und zweidimensionale Fingerprint-Methode überprüft.

Statt einer Ionenaustauschchromatographie in Gegenwart von Harnstoff oder Formamid kann man auch eine mehrfache Umkehrphasenchromatographie, eine Gelelektrophorese unter denaturierenden Bedingungen oder eine der vielen möglichen Kombinationen dieser Verfahren anwenden.

Herstellung der Massenspektrogramme der Fig. 1a bis 1c

Die Figuren 1a bis 1c zeigen die relevanten Regionen der Negativionen eines unter Beschuß mit schnellen Atomen durchgeführten Massenspektrogramme (FAB) von a = d(A-C-T-C-G-A-T-G), b = d(G-C-G-A-T-C-G-C) und c = d(G-A-A-G-A-T-C-T-T-C) unter Ausschluß der Glycerinmatrix. Die Massendifferenz zwischen zwei Marken ist ein Indiz für das jeweilige Nukleotid. Die Massendifferenzen oberhalb der Spektren betreffen Fragmentionen mit 5'-Phosphatenden (5'-Phosphatsequenzionen bzw. 5'-P-Sequenzionen) und die Massendifferenzen unterhalb der Spektren betreffen Fragmentionen mit 3'-Phosphatenden (3'-Phosphatsequenzionen bzw. 3'-P-Sequenzionen). Die höchste Masse liefert das (M-H)⁻-Ion. Das entsprechende doppelt geladene Ion wurde bei der halben Masse gefunden. Die Spektren wurden mit einem Kratos MS 50 S mit einem Hochfeldmagneten (Massenbereich etwa 3000 bei 8 kV) und einer Kratos-FAB-quelle aufgenommen. Die Atomkanone verwendete Xenon und lieferte einen Strahl neutraler Atome bei 8 bis 9 keV. Es wurde eine Lösung des Triäthylammoniumsalzes jedes Oligodesoxyribonukleotids (4 bis 5 µl mit einem Gehalt entsprechend 1 bis 1,5 oD $_{260}$; etwa 10 nmol) in die Glycerinmatrix (etwa 2 µl) auf einem FAB-Kupferprobenträger injiziert. Das Wasser wurde über eine Schleuse (direct insertion lock) entfernt und die Spektren wurden bei einer Magnetauflösungsrate von 300 s/Dekade aufgezeichnet.

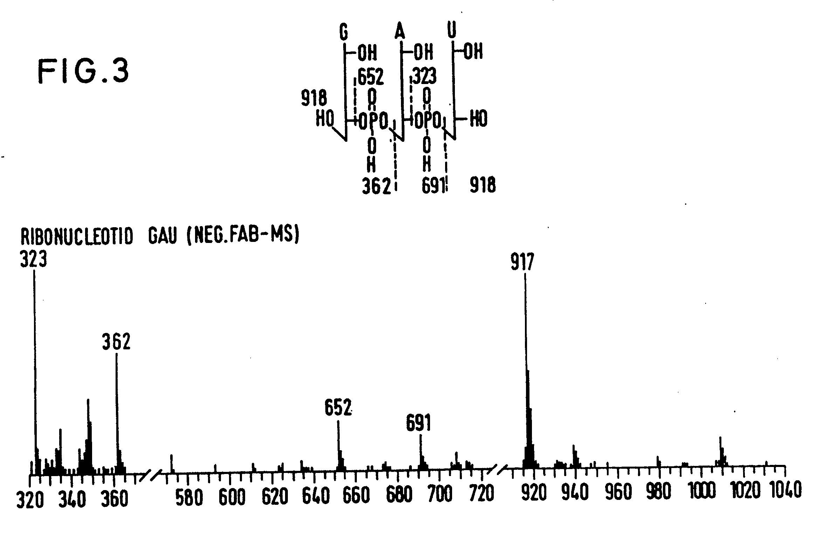

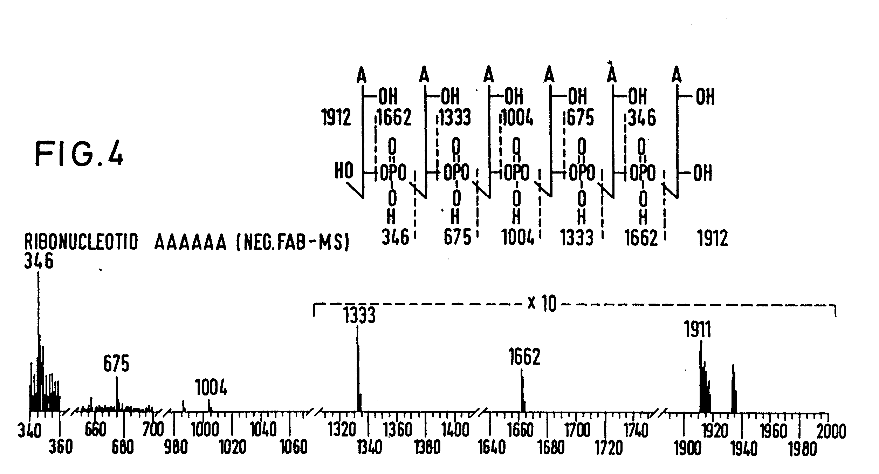

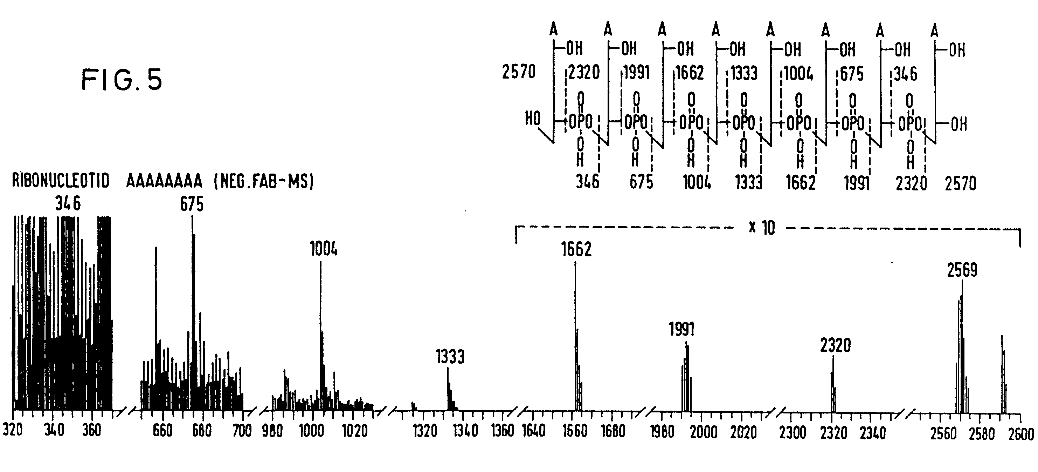

Das erfindungsgemäße Verfahren zur Sequenzanalyse von Oligoribonukleotiden ist in Tab. 3 und den Fig. 3 bis 5 erläutert.

1. Die Triethylammoniumsalze von Oligoribonukleotiden geben die zu den entsprechenden Oligodesoxyribonukleotiden analogen Massenpeaks (Beispiel: $rA_6$, $rA_8$).

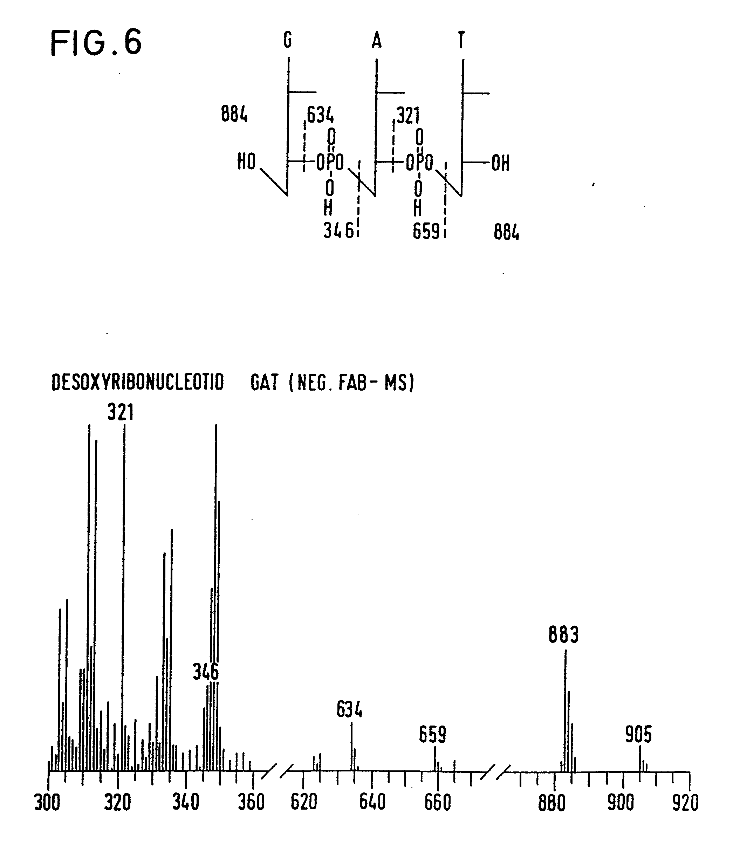

2. Die Analyse von gemischt-basigen Ribo-Tri- und Tetrameren zeigt, daß auch bei Oligoribonukleotiden die Ionen unterschiedlichen Typs jedoch mit der gleichen Anzahl von Nukleotideinheiten regelmäßig durch ihre Peakintensität zu unterscheiden sind (Beispiel: d(G-A-T) und r(G-A-U), wobei zu beachten ist, daß Oligoribonukleotide üblicherweise das Nukleotid Uridin (U) statt Thymidin (T) enthalten (vgl. Fig. 3 und 6).

Das Verfahren ist analog auch auf chemisch modifizierte Oligomere anwendbar, wobei entweder im zu untersuchenden Material mindestens eine Phosphatladung vorhanden sein muß (vgl. Tab. 4 und 5), oder eine solche unter den Meßbedingungen erzeugt werden kann. Letzteres ist der Fall bei Phosphatschutzgruppen, die reduktiv abspaltbar sind (Beispiel: Tribromethyl- oder Cyanoethyl-Schutzgruppe).

Eine besondere Bedeutung dieser Variante des erfindungsgemäßen Verfahrens liegt in der Möglichkeit, Zwischenstufen für die Oligonukleotidsynthese auf ihre Identität zu untersuchen. Es werden heutzutage meist teilgeschützte Mono- und Dinukleotide für die Synthese verwendet. Letztere entzogen sich bislang einer massenspektrometrischen Sequenzanalyse. Die Ergebnisse des erfindungsgemäßen Verfahrens sind in Tabelle 4 zusammengefaßt. Die charakteristischen Sets von Peaks ermöglichen eine eindeutige Sequenzaussage für Dinukleotide.

**Tab. 3:** Sequenz-Ionen von Oligoribonukleotiden

| Nucleosid | C | U | A | G |
|---|---|---|---|---|
| erstes Fragment (Nukleosid + $PO_3$) | 322 | 323 | 346 | 362 |
| Folgefragmente (Nukleosid + $PO_2-H$) | 305 | 306 | 329 | 345 |
| letztes Fragment (Nukleosid - OH) | 225 | 226 | 249 | 265 |

0 103 677

$$\text{DMTr} - \text{dX} - \text{p}_{ocp} - \text{dY} - \text{p}_{ocp} - \text{O}^-$$

$$\text{X} : \text{A}^{bz}, \text{C}^{bz}, \text{G}^{ib}, \text{T}$$

$$\text{Y} : \text{A}^{bz}, \text{C}^{bz}, \text{G}^{ib}, \text{T}$$

DMTr = Dimethoxytrityl
ocp = o-Chlorphenyl
X, Y = Nukleosid
bz = Benzoyl
ib = i-Butyryl
p = Phosphatrest

| X \ Y | A | C | G | T |
|---|---|---|---|---|
| **A** | M 1373<br>pAp 734<br>Ap 544<br>DMTrAp 846 | M 1349<br>pCp 710<br>Cp 520<br>DMTrAp 846 | M 1355<br>pGp 716<br>Gp 526<br>DMTrAp 846 | M 1260<br>pTp 621<br>Tp 431<br>DMTrAp 846 |
| **C** | M 1349<br>pAp 734<br>Ap 544<br>DMTrCp 822 | M 1325<br>pCp 710<br>Cp 520<br>DMTrCp 822 | M 1331<br>pGp 716<br>Gp 526<br>DMTrCp 822 | M 1236<br>pTp 621<br>Tp 431<br>DMTrCp 822 |
| **G** | M 1355<br>pAp 734<br>Ap 544<br>DMTrGp 828 | M 1331<br>pCp 710<br>Cp 520<br>DMTrGp 828 | M 1337<br>pGp 716<br>Gp 526<br>DMTrGp 828 | M 1242<br>pTp 621<br>Tp 431<br>DMTrGp 828 |
| **T** | M 1260<br>pAp 734<br>Ap 544<br>DMTrTp 733 | M 1236<br>pCp 710<br>Cp 520<br>DMTrTp 733 | M 1242<br>pGp 716<br>Gp 526<br>DMTrTp 733 | M 1147<br>pTp 621<br>Tp 431<br>DMTrTp 733 |

**Tab. 5:** Sequenz-Ionen von 5'-(MMTR)-Oligodeoxyribonukleotiden; MMTR = Monomethoxytrityl

| Nucleosid | erstes Fragment 3'-P | erstes Fragment 5'-P | Folgefragmente | letztes Fragment 3'-P | letztes Fragment 5'-P |
|---|---|---|---|---|---|
| G | 618 | 346 | 329 | 249 | 521 |
| A | 602 | 330 | 313 | 233 | 505 |
| T | 593 | 321 | 304 | 224 | 496 |
| C | 578 | 306 | 289 | 209 | 481 |

## Literatur

1. Berlin, Y.A., Chakhmakhcheva, O.G., Efimov, V.A., Kolosov, M.N. & Korobko, Y.G., Tetrahedron Lett. 1973, 1353 - 1354

2. Letsinger, R.L., Finnan, J.L., Heavner, G.A. & Lunsford, W.B., J. Am. Chem. Soc. 97, 3278 - 3279 (1975)

3. Reese, C.B., Titmas, R.C. & Yau, L., Tetrahedron Lett. 1978, 2727 -2730

4. Kohli, V., Blöcker, H. & Köster, H., Tetrahedron Lett. 21, 2683 -2686 (1980)

5. Matteucci, M.D. & Caruthers, M.H., Tetrahedron Lett. 1980, 719 - 722

6. Duckworth, M.L., Gait, M.J., Goelet, P., Hong, G.F., Singh, M. & Titmas, R.C., Nucl. Acids Res. 9, 1691 - 1706 (1981)

7. Ito, H., Ike, Y., Ikuta, S. & Itakura, K., Nucl. Acids Res. 10, 1755 -1769 (1982)

8. Maxam, A.M. & Gilbert, W., Proc. Natn. Acad. Sci. U.S.A. 74, 560 - 564 (1977)

9. Brownlee, G.G. & Sanger, F., Eur. J. Biochem. 11, 395-399 (1969)

10. Barber, M., Bordoli, R.S., Sedgwick, R.D. & Tyler, A.N., Nature 293, 270 -275 (1981)

11. Frank, R. & Köster, H., Nucl. Acids Res. 6, 2069 -2087 (1979)

12. McNeal, C.J., Ogilvie, K.K., Theriault, N.Y. & Nemer, M.J., J. Am. Chem. Soc. 104, 976 - 980 (1982)

13. Morris, R.M., Dell, A. & McDowell, R.A., Biomed. Mass Spectrometry 8, 463 - 473 (1981)

14. McNeal, C.J., Narang, S.A., Macfarlane, R.D., Hsuing, H.M. & Brousseau, R., Proc. Natl. Acad. Sci. USA 77 (2), 735 -739 (1980)

15. Ens, W., Standing, K.G., Westmore, J.B., Ogilvie, K.K. & Nemer, M.J., Anal. Chem. 54 (5), 960 - 966 (1982)

## Patentansprüche

1. Verfahren zur massenspektrometrischen Sequenzanalyse eines Oligoribonukleotids oder Oligodesoxyribonukleotids, dadurch gekennzeichnet, daß man
   (a) das Nukleotid ungeschützt der Massenspektrometrie unterwirft,
   (b) die Negativionen registriert und
   (c1) die Massendifferenz zwischen gewichtsmäßig aufeinanderfolgenden 5'-P-Ionen und/oder
   (c2) die Massendifferenz zwischen gewichtsmäßig aufeinanderfolgenden 3'-P-Ionen ermittelt und
   (d) die ermittelten aufeinanderfolgenden Massendifferenzen den Basen zuordnet.

2. Verfahren nach Anspruch 1, dadurch gekennzeichnet, daß man das Nukleotid einer FAB-Massenspektrometrie unterwirft.

3. Verfahren zur massenspektrometrischen Sequenzanalyse eines modifizierten Oligoribonukleotids oder Oligodesoxyribonukleotids, dadurch gekennzeichnet, daß man
   (a) das modifizierte Nukleotid einer FAB-Massenspektrometrie oder SIMS-Massenspektrometrie (ausgenommen Static-SIMS-Massenspektrometrie) unterwirft, wobei es sich bei dem modifizierten Nukleotid um ein Nukleotid mit mindestens einem ionischen Phosphatrest (Phosphatladung) oder um ein unter Meßbedingungen einen derartigen Phosphatrest lieferndes Nukleotid handelt,
   (b) die Negativionen registriert und
   (c1) die Massendifferenz zwischen gewichtsmäßig aufeinanderfolgenden 5'-P-Ionen und/oder
   (c2) die Massendifferenz zwischen gewichtsmäßig aufeinanderfolgenden 3'-P-Ionen ermittelt und
   (d) die ermittelten aufeinanderfolgenden Massendifferenzen den Basen zuordnet.

4. Verfahren nach Anspruch 1, 2 oder 3, dadurch gekennzeichnet, daß man ein Nukleotid mit einer Homogenität von 95, insbesondere 97 und vorzugsweise 99 % verwendet.

## Claims

1. Method for the mass spectrometric sequence analysis of an oligoribonucleotide or oligodeoxyribonucleotide, characterised in that
   (a) the unprotected nucleotide is subjected to mass spectrometry
   (b) the negative ions are recorded, and
   (c1) the difference in mass between successive 5'-P ions by weight and/or
   (c2) the difference in mass between successive 3'-P ions by weight is determined, and
   (d) the successive mass differences that have been determined are assigned to the bases.

2. Method according to claim 1, characterised in that the nucleotide is subjected to FAB mass spectrometry.

3. Method for the mass spectrometric sequence analysis of a modified oligoribonucleotide or oligodeoxyribonucleotide, characterised in that
   (a) the modified nucleotide is subjected to FAB mass spectrometry or SIMS mass spectrometry (except static SIMS mass spectrometry), the modified nucleotide being a nucleotide having at least one ionic phosphate radical (phosphate charge) or being a nucleotide that yields such a phosphate radical under measuring conditions,

(b) the negative ions are recorded, and
(c1) the difference in mass between successive 5'-P ions by weight and/or
(c2) the difference in mass between successive 3'-P ions by weight is determined, and
(d) the successive mass differences that have been determined are assigned to the bases.
4. Method according to claim 1, 2 or 3, characterised in that a nucleotide having a homogeneity of 95, especially 97 and preferably 99% is used.

**Revendications**

1. Procédé pour l'analyse par spectrométrie de masse des séquences d'un oligoribonucléotide ou d'un oligodésoxyribonucléotide, caractérisé en ce que:
(a) on soumet à une spectrométrie de masse le nucléotide non protégé,
(b) on enregistre les ions négatifs et
(c1) on détermine la différence des masses entre des ions deséquences P en 5' pondéralement successifs et/ou
(c2) on détermine la différence des masses entre des ions de séquences P en 3' pondéralement successifs, et
(d) on attribue aux bases des différences de masses successives ainsi déterminées.
2. Procédé selon la revendication 1, caractérisé en ce qu'on soumet le nucléotide à une spectrométrie de masse à l'aide d'un bombardement par des atomes rapides.
3. Procédé pour une analyse par spectrométrie de masse des séquences d'un oligoribonucléotide ou oligodésoxyribonucléotide modifié, procédé caractérisé en ce que:
(a) on soumet le nucléotide modifié à une spectrométrie de masse par bombardement par des atomes rapides ou à une spectrométrie de masse avec détermination du temps de vol d'ions secondaircs (à l'exclusion d'une spectrométrie de masse statique avec détermination du temps de vol d'ions secondaires), et il s'agit alors pour le nucléotide modifié d'un nucléotide comportant au moins un reste phosphate ionique (charge en phosphate) ou d'un nucléotide fournissant, dans des conditions de mesure, un tel reste phosphate,
(b) on enregistre les ions négatifs et
(c1) on détermine la différence de masse entre des ions de séquences P en 5' pondéralement successifs et/ou
(c2) on détermine la différence de masse entre des ions de séquences P en 3' pondéralement successifs, et
(d) on attribue aux bases des différences de masses successives ainsi déterminées.
4. Procédé selon la revendication 1, 2 ou 3, caractérisé en ce qu'on utilise un nucléotide ayant une homogénéité de 95, en particulier de 97 et avantageusement de 99 %.

pG    pT    pA    pG    pC.    pT    pC    pA

330  346  619    650  923  963    1292    1885  ×10

1854    2158  2174

1203,5  1212    1541  1581    2407

**FIG.1a**

Ap    Cp    Tp    Cp    Gp    Ap    Tp    G

0 103 677

pC    pG    pC    pT    pA    pG    pC    G

306    346  635    ×10
1870

924    2159    2199
964
1204  1228    2408
1277  1541  1581

**FIG.1b**

Gp    Cp    Gp    Ap    Tp    Cp    Gp    C

# FIG.1c

# FIG.2a

-H2407  2174  1885  1581  1292  963  650  346

A  C  T  C  G  A  T  G

HO—OPO—OPO—OPO—OPO—OPO—OPO—OPO—OH

OH  OH  OH  OH  OH  OH  OH

330  619  923  1212  1541  1854  2158  -H2407

# FIG.2b

-H2408  2159  1870  1541  1228  924  635  306

G  C  G  A  T  C  G  C

HO—OPO—OPO—OPO—OPO—OPO—OPO—OPO—OH

OH  OH  OH  OH  OH  OH  OH

346  635  964  1277  1581  1870  2199  -H2406

# FIG.2c

-H3024  2775  2462  2149  1820  1507  1203  914  610  306

G  A  A  G  A  T  C  T  T  C

HO—OPO—OPO—OPO—OPO—OPO—OPO—OPO—OPO—OH

OH  OH  OH  OH  OH  OH  OH  OH

346  659  972  1301  1614  1918  2207  2511  2815  -H3024

FIG.3

RIBONUCLEOTID GAU (NEG.FAB-MS)

FIG.4

RIBONUCLEOTID AAAAAA (NEG. FAB-MS)

FIG.5

RIBONUCLEOTID AAAAAAAA (NEG.FAB-MS)

# FIG.6

DESOXYRIBONUCLEOTID   GAT (NEG. FAB-MS)